(19) Europäisches Patentamt
European Patent Office
Office européen des brevets

(11) EP 1 030 848 B1

# (12) EUROPÄISCHE PATENTSCHRIFT

(45) Veröffentlichungstag und Bekanntmachung des Hinweises auf die Patenterteilung:
**02.05.2003 Patentblatt 2003/18**

(21) Anmeldenummer: **98951467.4**

(22) Anmeldetag: **25.09.1998**

(51) Int Cl.[7]: **C07D 249/12**, C07C 337/06

(86) Internationale Anmeldenummer:
**PCT/EP98/06113**

(87) Internationale Veröffentlichungsnummer:
**WO 99/018088 (15.04.1999 Gazette 1999/15)**

(54) **VERFAHREN ZUR HERSTELLUNG VON TRIAZOLINTHION-DERIVATEN**

METHOD FOR PRODUCING TRIAZOLINTHION DERIVATIVES

PROCEDE DE PREPARATION DE DERIVES DE THIONE DE TRIAZOLINE

(84) Benannte Vertragsstaaten:
**AT BE CH DE DK ES FR GB IT LI NL**

(30) Priorität: **08.10.1997 DE 19744400**

(43) Veröffentlichungstag der Anmeldung:
**30.08.2000 Patentblatt 2000/35**

(73) Patentinhaber: **Bayer CropScience AG**
**40789 Monheim (DE)**

(72) Erfinder:
- **JAUTELAT, Manfred**
  **D-51399 Burscheid (DE)**
- **HUPPERTS, Achim**
  **D-40217 Düsseldorf (DE)**
- **LANTZSCH, Reinhard**
  **D-42115 Wuppertal (DE)**

(56) Entgegenhaltungen:
**DE-A- 4 030 039** **DE-A- 4 339 412**

EP 1 030 848 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung betrifft ein neues Verfahren zur Herstellung von Triazolinthion-Derivaten, die als Wirkstoffe mit mikrobiziden, insbesondere fungiziden Eigenschaften bekannt sind.

Es ist bereits bekannt, daß sich Triazolinthion-Derivate herstellen lassen, indem man die entsprechenden Triazol-Derivate entweder nacheinander mit starken Basen und Schwefel umsetzt und dann hydrolysiert oder direkt mit Schwefel bei hohen Temperaturen umsetzt und dann mit Wasser behandelt (vgl. WO-A 96-16 048). Nachteilig an diesen Verfahren ist aber, daß die gewünschten Produkte nur in relativ niedrigen Ausbeuten anfallen, oder daß Umsetzungsbedingungen erforderlich sind, die im technischen Maßstab nur schwierig einzuhalten sind.

Weiterhin wurde schon beschrieben, daß sich bestimmte, in 3-Position substituierte 1,2,4-Triazolin-5-thione dadurch herstellen lassen, daß man N-Chlorthioformyl-N-(1-chloralk-1-en)-amine mit Carbonylhydrazin-Derivaten umsetzt (vgl. DE-A 197 01 032, DE-A 196 01 189 und EP-A 0 784 053). Die Synthese von entsprechenden Substanzen, die keinen Substituenten in der 3-Position enthalten, wird jedoch nicht erwähnt.

Ferner geht aus Bull. Chem. Soc. Japan 46, 2215 (1973) hervor, daß sich in 3-Position substituierte Triazolinthione synthetisieren lassen, indem man Phenylhydrazin mit Natriumthiocyanat und Ketonen oder Aldehyden in Gegenwart von Salzsäure umsetzt und die dabei anfallenden, in 3-Position substituierten Triazolidinthione mit oxidierenden Reagenzien behandelt. Beeinträchtigend an diesem Verfahren ist, daß sehr lange Reaktionszeiten erforderlich sind und keine in 3-Stellung unsubstituierten Triazolinthione auf diese Weise zugänglich sind.

Schließlich ist auch schon bekannt, daß man 2-(1-Chlor-cyclopropyl)-1-(2-chlorphenyl)-3-(1,2,4-triazol-1-yl)-propan-2-ol erhält, wenn man [1-(2-Chlor-phenyl)-2-(1-chlor-cyclopropyl-2-hydroxy]-propyl-1-hydrazin mit Formamidinacetat umsetzt (vgl. DE-A 40 30 039). Thiono-Derivate von Triazolen sind nach dieser Methode allerdings nicht zugänglich.

Es wurde nun gefunden, daß sich Triazolinthion-Derivate der Formel

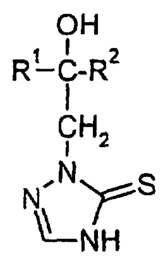

(I)

in welcher

$R^1$ für geradkettiges oder verzweigtes Alkyl mit 1 bis 4 Kohlenstoffatomen steht, wobei diese Reste einfach bis vierfach, gleichartig oder verschieden substituiert sein können durch Fluor, Chlor, Brom, Methoxy, Ethoxy, Propoxy, Isopropoxy, Alkoximino mit 1 oder 2 Kohlenstoffatomen im Alkoxyteil, Cyclopropyl, Cyclobutyl, Cyclopentyl und/oder Cyclohexyl, oder

für geradkettiges oder verzweigtes Alkenyl mit 2 bis 5 Kohlenstoffatomen steht, wobei jeder dieser Reste einfach bis dreifach, gleichartig oder verschieden substituiert sein kann durch Fluor, Chlor, Brom, Methoxy, Ethoxy, Propoxy, Isopropoxy, Cyclopropyl, Cyclobutyl, Cyclopentyl und/oder Cyclohexyl

oder

für Cycloalkyl mit 3 bis 6 Kohlenstoffatomen steht, wobei jeder dieser Reste einfach bis dreifach, gleichartig oder verschieden substituiert sein kann durch Fluor, Chlor, Brom, Cyano, Methyl, Ethyl, Propyl, Isopropyl und/oder tert. -Butyl,

oder

für Phenylalkyl mit 1 bis 4 Kohlenstoffatomen im geradkettigen oder verzweigten Alkylteil steht, wobei der Phenylteil einfach bis dreifach, gleichartig oder verschieden substituiert sein kann durch Fluor, Chlor, Brom, Methyl, Ethyl, tert. -Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,

oder

für Phenylalkenyl mit 2 bis 4 Kohlenstoffatomen im Alkenylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano, oder

für Phenoxyalkyl mit 1 bis 4 Kohlenstoffatomen im geradkettigen oder verzweigten Oxyalkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,

oder

für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,

und $R^2$ für geradkettiges oder verzweigtes Alkyl mit 1 bis 4 Kohlenstoffatomen steht, wobei diese Reste einfach bis vierfach, gleichartig oder verschieden substituiert sein können durch Fluor, Chlor, Brom, Methoxy, Ethoxy, Propoxy, Isopropoxy, Alkoximino mit 1 oder 2 Kohlenstoffatomen im Alkoxyteil, Cyclopropyl, Cyclobutyl, Cyclopentyl und/oder Cyclohexyl,

oder

für geradkettiges oder verzweigtes Alkenyl mit 2 bis 5 Kohlenstoffatomen steht, wobei jeder dieser Reste einfach bis dreifach, gleichartig oder verschieden substituiert sein kann durch Fluor, Chlor, Brom, Methoxy, Ethoxy, Propoxy, Isopropoxy, Cyclopropyl, Cyclobutyl, Cyclopentyl und/oder Cyclohexyl,

oder

für Cycloalkyl mit 3 bis 6 Kohlenstoffatomen steht, wobei jeder dieser Reste einfach bis dreifach, gleichartig oder verschieden substituiert sein kann durch Fluor, Chlor, Brom, Cyano, Methyl, Ethyl, Propyl, Isopropyl und/oder tert. -Butyl,

oder

für Phenylalkyl mit 1 bis 4 Kohlenstoffatomen im geradkettigen oder verzweigten Alkylteil steht, wobei der Phenylteil einfach bis dreifach, gleichartig oder verschieden substituiert sein kann durch Fluor, Chlor, Brom, Methyl, Ethyl, tert. -Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,

oder

für Phenylalkenyl mit 2 bis 4 Kohlenstoffatomen im Alkenylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,

oder

für Phenoxyalkyl mit 1 bis 4 Kohlenstoffatomen im geradkettigen oder verzweigten Oxyalkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,

oder

für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano, stehen,

herstellen lassen, indem man

a) in einer ersten Stufe Hydrazin-Derivate der Formel

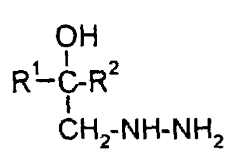

(II)

in welcher

$R^1$ und $R^2$ die oben angegebenen Bedeutungen haben, oder deren Additionssalze mit Chlorwasserstoff oder Schwefelsäure,

mit Thiocyanat der Formel

Y-SCN (III)

in welcher

Y für Natrium, Kalium oder Ammonium steht,

in Gegenwart eines Verdünnungsmittels und gegebenenfalls in Gegenwart eines Katalysators umsetzt und

b) die entstandenen Thiosemicarbazid-Derivate der Formel

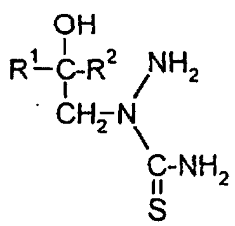

(IV)

in welcher
$R^1$ und $R^2$ die oben angegebenen Bedeutungen haben,
mit Ameisensäure, gegebenenfalls in Gegenwart eines Katalysators und gegebenenfalls in Gegenwart eines Verdünnungsmittels umsetzt.

Es ist als ausgesprochen überraschend zu bezeichnen, daß sich Triazolinthion-Derivate der Formel (I) nach dem erfindungsgemäßen Verfahren in wesentlich höheren Ausbeuten oder unter erheblich einfacheren Bedingungen herstellen lassen als nach den bisher bekannten Methoden. Unerwartet ist auch, daß bei der Durchführung der ersten Stufe des erfindungsgemäßen Verfahrens der Angriff des Thiocyanats in überwiegendem Maße nicht am terminalen, sondern am substituierten Stickstoffatom des Hydrazin-Derivates der Formel (II) erfolgt und somit das gewünschte Isomere mit hoher Selektivität entsteht.

Das erfindungsgemäße Verfahren zeichnet sich durch eine Reihe von Vorteilen aus. So ermöglicht es, wie schon erwähnt, die Synthese von Triazolinthion-Derivaten der Formel (I) in hoher Ausbeute. Günstig ist auch, daß die benötigten Ausgangsstoffe und Reaktionskomponenten einfach herstellbar und auch in größeren Mengen verfügbar sind. Ein weiterer Vorteil besteht schließlich darin, daß die Durchführung der einzelnen Reaktionsschritte und die Isolierung der Reaktionsprodukte keinerlei Schwierigkeiten bereitet.

Verwendet man 2-(1-Chlor-cyclopropyl)-3-(2-chlor-phenyl)-2-hydroxy-propyl-1-hydrazonium-sulfat als Ausgangssubstanz und setzt diese in der ersten Stufe mit Ammoniumthiocyanat um und läßt das dabei anfallende Thiosemicarbazid in der zweiten Stufe mit Ameisensäure reagieren, so kann der Verlauf des erfindungsgemäßen Verfahrens durch das folgende Formelschema veranschaulicht werden.

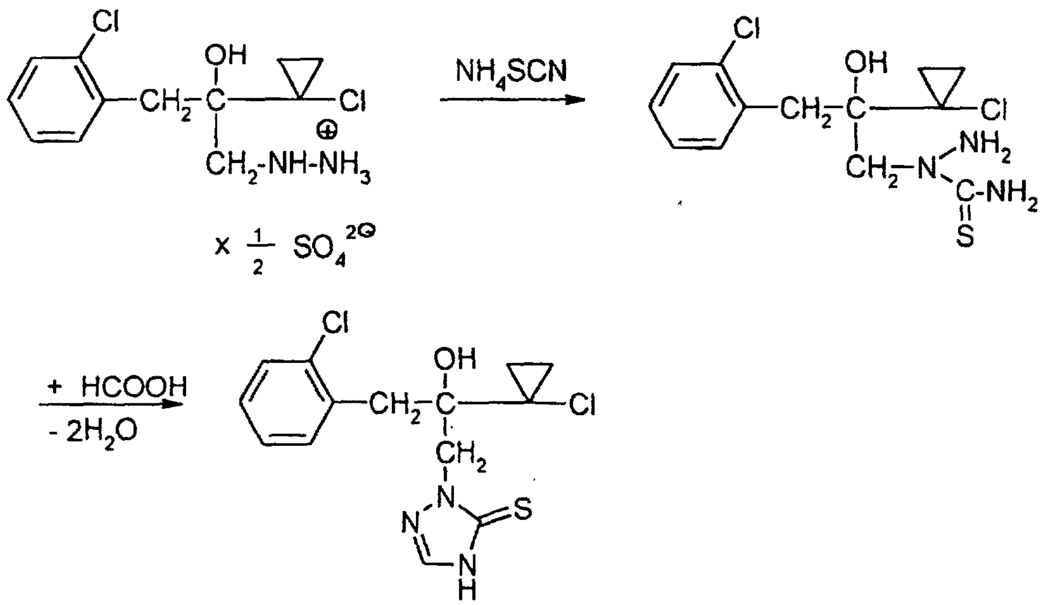


Die bei der Durchführung des erfindungsgemäßen Verfahrens als Ausgangsstoffe benötigten Hydrazin-Derivate sind durch die Formel (II) allgemein definiert.

Ganz besonders bevorzugt einsetzbar sind Hydrazin-Derivate der Formel (II), in welcher

$R^1$ bevorzugt für n-Propyl, Isopropyl, n-Butyl, i-Butyl, sek.-Butyl oder tert.-Butyl steht, wobei diese Reste einfach bis vierfach, gleichartig oder verschieden substituiert sein können durch Fluor, Chlor, Brom, Methoxy, Ethoxy, Propoxy, Isopropoxy, Methoximino, Ethoximino, Cyclopropyl, Cyclobutyl, Cyclopentyl und/oder Cyclohexyl,
oder
für geradkettiges oder verzweigtes Alkenyl mit 2 bis 5 Kohlenstoffatomen steht, wobei jeder dieser Reste einfach bis dreifach, gleichartig oder verschieden substituiert sein kann durch Fluor, Chlor, Brom, Methoxy, Ethoxy, Propoxy, Isopropoxy, Cyclopropyl, Cyclobutyl, Cyclopentyl und/oder Cyclohexyl,
oder
für 1-Methyl-cyclohexyl, Cyclohexyl, 1-Chlor-cyclopropyl, 1-Fluor-cyclopropyl, 1-Methyl-cyclopropyl, 1-Cyano-Cyclopropyl, Cyclopropyl, 1-Methyl-cyclopentyl oder 1-Ethyl-cyclopentyl steht,
oder
für Phenylalkyl mit 1 oder 2 Kohlenstoffatomen im geradkettigen oder verzweigten Alkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenylalkenyl mit 2 bis 4 Kohlenstoffatomen im Alkenylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenoxyalkyl mit 1 bis 4 Kohlenstoffatomen im geradkettigen oder verzweigten Oxyalkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio,

Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano, und

$R^2$ für n-Propyl, Isopropyl, n-Butyl, i-Butyl, sek.-Butyl oder tert.-Butyl steht, wobei diese Reste einfach bis vierfach, gleichartig oder verschieden substituiert sein können durch Fluor, Chlor, Brom, Methoxy, Ethoxy, Propoxy, Isopropoxy, Methoximino, Ethoximino, Cyclopropyl, Cyclobutyl, Cyclopentyl und/oder Cyclohexyl,

oder

für geradkettiges oder verzweigtes Alkenyl mit 2 bis 5 Kohlenstoffatomen steht, wobei jeder dieser Reste einfach bis dreifach, gleichartig oder verschieden substituiert sein kann durch Fluor, Chlor, Brom, Methoxy, Ethoxy, Propoxy, Isopropoxy, Cyclopropyl, Cyclobutyl, Cyclopentyl und/oder Cyclohexyl,

oder

für 1-Methyl-cyclohexyl, Cyclohexyl, 1-Chlor-cyclopropyl, 1-Fluor-cyclopropyl, 1-Methyl-cyclopropyl, 1-Cyano-cyclopropyl, Cyclopropyl, 1-Methyl-cyclopentyl oder 1-Ethyl-cyclopentyl steht,

oder

für Phenylalkyl mit 1 oder 2 Kohlenstoffatomen im geradkettigen oder verzweigten Alkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,

oder

für Phenylalkenyl mit 2 bis 4 Kohlenstoffatomen im Alkenylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,

oder

für Phenoxyalkyl mit 1 bis 4 Kohlenstoffatomen im geradkettigen oder verzweigten Oxyalkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,

oder

für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano.

Bevorzugt einsetzbar sind auch Additionssalze aus den zuvor genannten bevorzugten bzw. besonders bevorzugten Hydrazin-Derivaten der Formel (II) und Chlorwasserstoff oder Schwefelsäure. Bevorzugt in Frage kommen dabei Hydrazonium-Salze der Formel

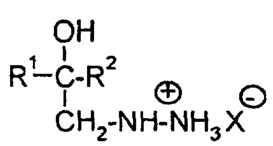

(IIa)

in welcher

$R^1$ und $R^2$ die oben angegebenen Bedeutungen haben und

$X^{\ominus}$ für Chlorid oder ein Äquivalent eines Sulfat-Anions steht.

Die Hydrazin-Derivate der Formel (II) sind bekannt oder lassen sich nach prinzipiell bekannten Verfahren herstellen (vgl. DE-A 40 30 039).

So erhält man Hydrazin-Derivate der Formel (II), indem man 1-Chlor-2-hydroxyethan-Derivate der Formel

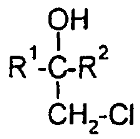

(V)

in welcher

$R^1$ und $R^2$ die oben angegebenen Bedeutungen haben,

mit Hydrazinhydrat gegebenenfalls in Gegenwart eines Verdünnungsmittels umsetzt.

Die Hydrazin-Derivate der Formel (II) lassen sich in ihre Säureadditions-Salze überführen, indem man sie mit einer anorganischen oder organischen Säure in Gegenwart eines Verdünnungsmittels umsetzt.

Die 1-Chlor-2-hydroxy-ethan-Derivate der Formel (V) sind bekannt oder lassen sich nach prinzipiell bekannten Verfahren herstellen (vgl. DE-A 40 30 039 und EP-A 0 297 345).

Als Verdünnungsmittel kommen bei dem obigen Verfahren zur Herstellung von Hydrazin-Derivaten der Formel (II) alle üblichen inerten, organischen Solventien in Frage. Vorzugsweise verwendbar sind Alkohole, wie Methanol, Ethanol oder n-Butanol, ferner Ether, wie Dioxan oder Methyl-tert.-butylether, und weiterhin aromatische Kohlenwasserstoffe, wie Benzol, Toluol oder Xylol. Es ist aber auch möglich, ohne zusätzliches Lösungsmittel zu arbeiten. In diesem Fall setzt man Hydrazinhydrat im Überschuß ein, so daß es sowohl als Reaktionskomponente als auch als Verdünnungsmittel fungiert.

Die Reaktionstemperaturen können bei der Herstellung von Hydrazin-Derivaten der Formel (II) nach dem obigen Verfahren in einem bestimmten Bereich variiert werden.

Im allgemeinen arbeitet man bei Temperaturen zwischen 60°C und 120°C, vorzugsweise zwischen 70°C und 110°C.

Bei der Herstellung von Hydrazin-Derivaten der Formel (II) nach dem obigen Verfahren setzt man auf 1 Mol an 1-Chlor-2-hydroxy-ethan-Derivat der Formel (V) im allgemeinen 1 bis 20 Mol, vorzugsweise 5 bis 15 Mol an Hydrazinhydrat ein. Die Aufarbeitung erfolgt nach üblichen Methoden. Im allgemeinen verfährt man in der Weise, daß man das Reaktionsgemisch mit einem mit Wasser wenig mischbaren organischen Solvens, wie Methyl-tert.-butylether oder Toluol versetzt, die wäßrige Phase abtrennt, die organische Phase wäscht und trocknet.

Bei der Überführung der Hydrazin-Derivate der Formel (II) in ihre Säureadditions-Salze kommen als Verdünnungsmittel alle für derartige Umsetzungen üblichen organischen Solventien in Betracht. Vorzugsweise verwendbar sind aromatische Kohlenwasserstoffe, wie Benzol, Toluol oder Xylol, oder Ether, wie Dioxan oder Methyltert.-butylether.

Bei der Überführung von Hydrazin-Derivaten der Formel (II) in Salze arbeitet man im allgemeinen bei Raumtemperatur. Es ist aber auch möglich bei etwas erhöhter oder niedrigerer Temperatur zu arbeiten.

Bei der Überführung von Hydrazin-Derivaten der Formel (II) in Salze geht man im allgemeinen so vor, daß man Hydrazin-Derivat der Formel (II) in einem Solvens löst und mit einer äquivalenten Menge oder einem Überschuß an Säure versetzt. Bei der Herstellung von Chloriden kann entweder Salzsäure oder gasförmiger Chlorwasserstoff verwendet werden. Die Salze lassen sich nach üblichen Methoden isolieren.

Die bei der Durchführung der ersten Stufe des erfindungsgemäßen Verfahrens als Reaktionskomponenten benötigten Thiocyanate der Formel (III) sind bekannt.

Als Verdünnungsmittel kommen bei der Durchführung der ersten Stufe des erfindungsgemäßen Verfahrens alle für derartige Umsetzungen üblichen, inerten organischen Solventien in Betracht. Vorzugsweise verwendbar sind aromatische Kohlenwasserstoffe, wie Benzol, Toluol oder Xylol, ferner Ether, wie Dioxan oder Methyltert.-butylether, und weiterhin Ester, wie Essigsäureethylester.

Als Katalysatoren kommen bei der Durchführung der ersten Stufe des erfindungsgemäßen Verfahrens alle für derartige Umsetzungen üblichen Reaktionsbeschleuniger in Frage. Vorzugsweise verwendbar ist Wasser.

Die Reaktionstemperaturen können bei der Durchführung der ersten Stufe des erfindungsgemäßen Verfahrens in einem größeren Bereich variiert werden. Im allgemeinen arbeitet man bei Temperaturen zwischen 50°C und 120°C, vorzugsweise zwischen 70°C und 110°C.

Sowohl bei der Durchführung der ersten als auch der zweiten Stufe des erfindungsgemäßen Verfahrens arbeitet man im allgemeinen unter Atmosphärendruck. Es ist aber auch möglich, unter erhöhtem Druck oder, sofern keine gasförmigen Komponenten an der Reaktion beteiligt sind, auch unter vermindertem Druck zu arbeiten.

Bei der Durchführung der ersten Stufe des erfindungsgemäßen Verfahrens setzt man auf 1 Mol an Hydrazin-Derivat der Formel (II) im allgemeinen 1 bis 2 Mol an Thiocyanat der Formel (III) sowie gegebenenfalls eine geringe Menge an Katalysator ein. Die Aufarbeitung erfolgt nach üblichen Methoden. Im allgemeinen geht man so vor, daß man das Reaktionsgemisch mit Wasser wäscht, die organische Phase trocknet und einengt und den verbleibenden Rückstand nach üblichen Methoden, z.B. durch Chromatographie oder Umkristallisation, von unerwünschten Bestandteilen befreit.

Die bei der Durchführung der zweiten Stufe des erfindungsgemäßen Verfahrens als Ausgangsstoffe benötigten Thiosemicarbazid-Derivate sind durch die Formel (IV) allgemein definiert. In dieser Formel haben $R^1$ und $R^2$ vorzugsweise diejenigen Bedeutungen, die bereits im Zusammenhang mit der Beschreibung der Hydrazin-Derivate der Formel (II) vorzugsweise für diese Reste genannt wurden.

Die Thiosemicarbazid-Derivate der Formel (IV) sind bisher noch nicht bekannt. Sie lassen sich durch die Umsetzung in der ersten Stufe des erfindungsgemäßen Verfahrens herstellen.

Als Katalysatoren kommen bei der Durchführung der zweiten Stufe des erfindungsgemäßen Verfahrens alle für derartige Umsetzungen üblichen Reaktionsbeschleuniger in Frage. Vorzugsweise verwendbar sind Säuren, wie Salzsäure oder Schwefelsäure, und ferner Metalloxide, wie amorphes Titandioxid.

Als Verdünnungsmittel kommen bei der Durchführung der zweiten Stufe des erfindungsgemäßen Verfahrens alle für derartige Umsetzungen üblichen, schwach polaren organischen Solventien in Betracht. Vorzugsweise verwendbar sind Ester, wie Essigsäureethylester oder Ameisensäure-isobutylester, und auch Ameisensäure.

Die Reaktionstemperaturen können bei der Durchführung der zweiten Stufe des erfindungsgemäßen Verfahrens innerhalb eines bestimmten Bereiches variiert werden. Im allgemeinen arbeitet man bei Temperaturen zwischen 80°C und 120°C, vorzugsweise zwischen 90°C und 110°C.

Bei der Durchführung der zweiten Stufe des erfindungsgemäßen Verfahrens setzt man auf 1 Mol an Thiosemicarbazid-Derivat der Formel (IV) einen Überschuß, im allgemeinen 5 bis 15 Mol an Ameisensäure sowie gegebenenfalls eine geringe Menge an Katalysator ein. Die Aufarbeitung erfolgt nach üblichen Methoden. Im allgemeinen verfährt man in der Weise, daß man das Reaktionsgemisch gegebenenfalls nach vorherigem Verdünnen mit einem mit Wasser wenig mischbaren organischen Solvens, mit wäßriger Salzlösung ausschüttelt, die organische Phase trocknet und einengt. Gegebenenfalls dann noch vorhandene Verunreinigungen lassen sich nach üblichen Methoden, wie Umkristallisation oder Chromatographie, abtrennen.

In einer besonderen Variante läßt sich das erfindungsgemäße Verfahren in der Weise durchführen, daß man 1-Chlor-2-hydroxy-ethan-Derivate der Formel (V) mit Hydrazinhydrat umsetzt und die dabei entstehenden Hydrazin-Derivate der Formel (II) dann ohne vorherige Isolierung weiter umsetzt. Demgemäß lassen sich Triazolinthione der Formel (I) auch dadurch herstellen, daß man

- 1-Chlor-2-hydroxy-ethan-Derivate der Formel

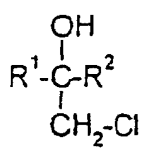

(V)

in welcher
$R^1$ und $R^2$ die oben angegebenen Bedeutungen haben,
mit Hydrazinhydrat gegebenenfalls in Gegenwart eines Verdünnungsmittels umsetzt und die dabei entstehenden Hydrazin-Derivate der Formel

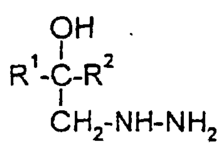

(II)

in welcher
$R^1$ und $R^2$ die oben angegebenen Bedeutungen haben, gegebenenfalls mit Chlorwasserstoff oder Schwefelsäure in Gegenwart eines Verdünnungsmittels umsetzt,

- und die Hydrazin-Derivate der Formel (II) oder deren Additions-Salze mit Chlorwasserstoff oder Schwefelsäure ohne vorherige Isolierung mit Thiocyanat der Formel

Y-SCN (III)

in welcher

Y für Natrium, Kalium oder Ammonium steht,

in Gegenwart eines Verdünnungsmittels und gegebenenfalls in Gegenwart eines Katalysators umsetzt und

- die entstandenen Thiosemicarbazid-Derivate der Formel

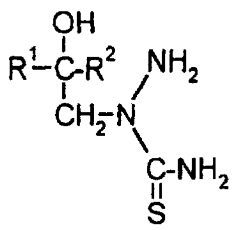

(IV)

in welcher
$R^1$ und $R^2$ die oben angegebenen Bedeutungen haben,
mit Ameisensäure, gegebenenfalls in Gegenwart eines Katalysators und gegebenenfalls in Gegenwart eines Verdünnungsmittels umsetzt.

Bei der Durchführung der einzelnen Stufen dieses Verfahrens geht man in der schon oben beschriebenen Weise vor.

Die erfindungsgemäß herstellbaren Triazolinthion-Derivate können in der "Thiono"-Form der Formel

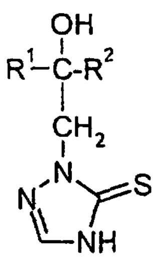

(I)

oder in der tautomeren "Mercapto"-Form der Formel

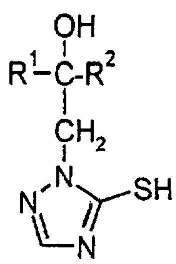

(Ia)

vorliegen.

Der Einfachheit halben wird jeweils nur die "Thiono"-Form aufgeführt.

Die erfindungsgemäß herstellbaren Triazolinthion-Derivate sind als Wirkstoffe mit mikrobiziden, insbesondere

fungiziden Eigenschaften bekannt (vgl. WO-A 96-16 048).

Die Durchführung des erfindungsgemäßen Verfahrens wird durch die folgenden Beispiele veranschaulicht.

## Herstellungsbeispiele

### Beispiel 1

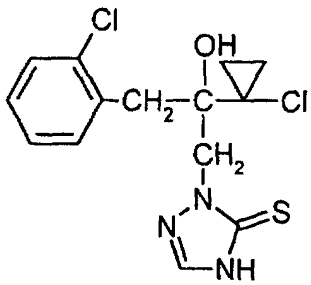

a) Herstellung der Verbindung der Formel

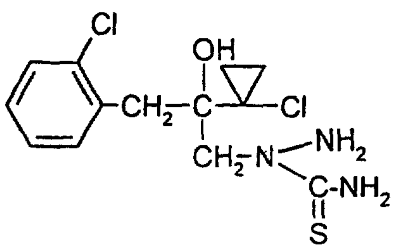

(IV-1)

Ein Gemisch aus 4,86 g (15 mmol) 2-(1-Chlor-cyclopropyl)-3-(2-chlor-phenyl)-2-hydroxy-propyl-1-hydraziniumsulfat, 1,26 g (16,5 mmol) Ammoniumthiocyanat und 30 ml Essigsäureethylester wird 3 Stunden lang unter Rühren auf 74 bis 76°C erhitzt. Danach läßt man das Reaktionsgemisch auf Raumtemperatur abkühlen und wäscht dann mit 20 ml Wasser. Die organische Phase wird über Natriumsulfat getrocknet, filtriert und unter vermindertem Druck eingeengt. Man erhält auf diese Weise 5,81 g eines Produktes, das gemäß HPLC-Analyse zu 65,9 % aus dem Thiosemicarbazid-Derivat der Formel (IV-1) besteht. Die Ausbeute errechnet sich danach zu 76,4 % der Theorie. Nach Umkristallisation aus Methanol fällt das Thiosemicarbazid-Derivat der Formel (IV-1) in Form eines kristallinen Festproduktes an, das bei 128 bis 129°C schmilzt.

b) Herstellung der Verbindung der Formel

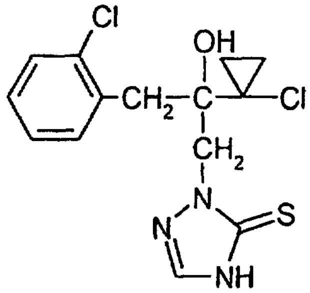

Ein Gemisch aus 3,35 g (10 mmol) Thiosemicarbazid-Derivat der Formel (IV-1) in 10 ml Ameisensäure-isobutylester wird bei Raumtemperatur unter Rühren mit 5 ml (132 mmol) Ameisensäure versetzt und dann 5,5 Stunden auf 95°C erhitzt. Nach dem anschließenden Abkühlen auf Raumtemperatur verdünnt man das Reaktionsgemisch mit 50 ml Essigsäureethylester und wäscht dreimal mit je 10 ml gesättigter, wäßriger Ammoniumchlorid-Lösung. Die organische Phase wird über Natriumsulfat getrocknet, filtriert und unter vermindertem Druck eingeengt. Man erhält auf diese Weise 3,65 g eines Produktes, das gemäß HPLC-Analyse zu 86,4 % aus 2-(1-Chlor-cyclopropyl)-1-(2-chlor-phenyl)-3-(4,5-dihydro-1,2,4-triazol-5-thiono-1-yl)-propan-2-ol besteht. Die Ausbeute errechnet sich danach zu 91,6 % der Theorie.

**Vergleichsbeispiele**

**Beispiel A**

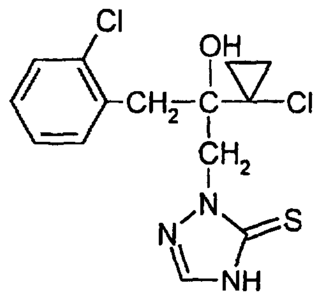

Ein Gemisch aus 3,12g (10 mMol) 2-(1-Chlor-cyclopropyl)-1-(2-chlorphenyl)-3-(1,2,4-triazol-1-yl)-propan-2-ol und 45 ml absolutem Tetrahydrofuran wird bei -20°C mit 8,4 ml (21 mMol) n-Butyl-lithium in Hexan versetzt und 30 Minuten bei 0°C gerührt. Danach wird das Reaktionsgemisch auf -70°C abgekühlt, mit 0,32 g (10 mMol) Schwefel-Pulver versetzt und 30 Minuten bei -70°C gerührt. Es wird auf -10°C erwärmt, mit Eiswasser versetzt und durch Zugabe von verdünnter Schwefelsäure auf einen pH-Wert von 5 eingestellt. Man extrahiert mehrfach mit Essigsäureethylester, trocknet die vereinigten organischen Phasen über Natriumsulfat und engt unter vermindertem Druck ein. Man erhält auf diese Weise 3,2 g eines Produktes, das gemäß gaschromatografischer Analyse zu 95 % aus 2-(1-Chlor-cyclopropyl)-1-(2-chlor-phenyl)-3-(4,5-dihydro-1,2,4-triazol-5-thiono-1-yl)-propan-1-ol besteht. Nach Umkristallisation aus Toluol erhält man diese Substanz als Feststoff, der bei 138 bis 139°C schmilzt.

**Beispiel B**

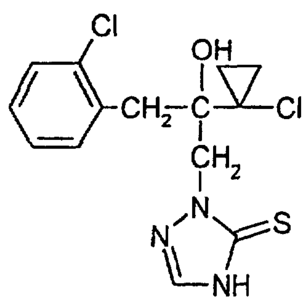

Ein Gemisch aus 3,12 g (10 mmol) 2-(1-Chlor-cyclopropyl)-1-(2-chlor-phenyl)-3-(1,2,4-triazol-1-yl)-propan-2-ol, 0,96 g (30 mmol) Schwefel-Pulver und 20 ml absolutem N-Methyl-pyrrolidon wird unter Rühren 44 Stunden auf 200°C erhitzt. Anschließend wird das Reaktionsgemisch unter vermindertem Druck (0,2 mbar) eingeengt. Das dabei anfallende Rohprodukt (3,1 g) wird aus Toluol umkristallisiert. Man erhält auf diese Weise 0,7 g (20 % der Theorie) an 2-(1-Chlor-cyclopropyl)-1-(2-chlor-phenyl)-3-(4,5-dihydro-1,2,4-triazol-5-thiono-1-yl)-propan-2-ol in Form einer Festsubstanz, die bei 138 bis 139°C schmilzt.

## Patentansprüche

1. Verfahren zur Herstellung von Triazolinthion-Derivaten der Formel

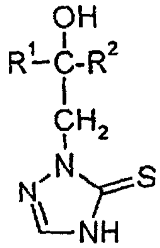

(I)

in welcher

$R^1$ für geradkettiges oder verzweigtes Alkyl mit 1 bis 4 Kohlenstoffatomen steht, wobei diese Reste einfach bis vierfach, gleichartig oder verschieden substituiert sein können durch Fluor, Chlor, Brom, Methoxy, Ethoxy, Propoxy, Isopropoxy, Alkoximino mit 1 oder 2 Kohlenstoffatomen im Alkoxyteil, Cyclopropyl, Cyclobutyl, Cyclopentyl und/oder Cyclohexyl,
oder
für geradkettiges oder verzweigtes Alkenyl mit 2 bis 5 Kohlenstoffatomen steht, wobei jeder dieser Reste einfach bis dreifach, gleichartig oder verschieden substituiert sein kann durch Fluor, Chlor, Brom, Methoxy, Ethoxy, Propoxy, Isopropoxy, Cyclopropyl, Cyclobutyl, Cyclopentyl und/oder Cyclohexyl
oder
für Cycloalkyl mit 3 bis 6 Kohlenstoffatomen steht, wobei jeder dieser Reste einfach bis dreifach, gleichartig oder verschieden substituiert sein kann durch Fluor, Chlor, Brom, Cyano, Methyl, Ethyl, Propyl, Isopropyl und/oder tert.-Butyl,
oder
für Phenylalkyl mit 1 bis 4 Kohlenstoffatomen im geradkettigen oder verzweigten Alkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenylalkenyl mit 2 bis 4 Kohlenstoffatomen im Alkenylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenoxyalkyl mit 1 bis 4 Kohlenstoffatomen im geradkettigen oder verzweigten Oxyalkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthlo, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
und

$R^2$ für geradkettiges oder verzweigtes Alkyl mit 1 bis 4 Kohlenstoffatomen steht, wobei diese Reste einfach bis

vierfach, gleichartig oder verschieden substituiert sein können durch Fluor, Chlor, Brom, Methoxy, Ethoxy, Propoxy, Isopropoxy, Alkoximino mit 1 oder 2 Kohlenstoffatomen im Alkoxyteil, Cyclopropyl, Cyclobutyl, Cyclopentyl und/oder Cyclohexyl,
oder
für geradkettiges oder verzweigtes Alkenyl mit 2 bis 5 Kohlenstoffatomen steht, wobei jeder dieser Reste einfach bis dreifach, gleichartig oder verschieden substituiert sein kann durch Fluor, Chlor, Brom, Methoxy, Ethoxy, Propoxy, Isopropoxy, Cyclopropyl, Cyclobutyl, Cyclopentyl und/oder Cyclohexyl,
oder
für Cycloalkyl mit 3 bis 6 Kohlenstoffatomen steht, wobei jeder dieser Reste einfach bis dreifach, gleichartig oder verschieden substituiert sein kann durch Fluor, Chlor, Brom, Cyano, Methyl, Ethyl, Propyl, Isopropyl und/oder tert.-Butyl,
oder
für Phenylalkyl mit 1 bis 4 Kohlenstoffatomen im geradkettigen oder verzweigten Alkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenylalkenyl mit 2 bis 4 Kohlenstoffatomen im Alkenylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenoxyalkyl mit 1 bis 4 Kohlenstoffatomen im geradkettigen oder verzweigten Oxyalkylteil steht, wobei der Phenylteil einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,
oder
für Phenyl steht, das einfach bis dreifach, gleichartig oder verschieden substituiert sein kann durch Fluor, Chlor, Brom, Methyl, Ethyl, tert.-Butyl, Methoxy, Ethoxy, Methylthio, Trifluormethyl, Trifluormethoxy, Trifluormethylthio, Chlordifluormethoxy, Difluormethoxy, Chlordifluormethylthio, Methoxycarbonyl, Ethoxycarbonyl, Methoximinomethyl, 1-Methoximinoethyl, Nitro und/oder Cyano,

**dadurch gekennzeichnet, daß** man

a) in einer ersten Stufe Hydrazin-Derivate der Formel

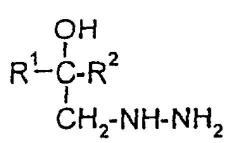

(II)

in welcher
$R^1$ und $R^2$ die oben angegebenen Bedeutungen haben,
oder deren Additionssalze mit Chlorwasserstoff oder Schwefelsäure,
mit Thiocyanat der Formel

Y-SCN (III)

in welcher

Y für Natrium, Kalium oder Ammonium steht,

in Gegenwart eines Verdünnungsmittels und gegebenenfalls in Gegenwart eines Katalysators umsetzt und

b) die entstandenen Thiosemicarbazid-Derivate der Formel

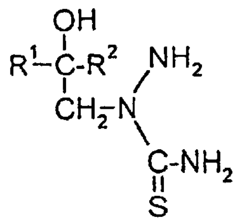

(IV)

in welcher
$R^1$ und $R^2$ die oben angegebenen Bedeutungen haben,
mit Ameisensäure, gegebenenfalls in Gegenwart eines Katalysators und gegebenenfalls in Gegenwart eines Verdünnungsmittels umsetzt.

**2.** Verfahren gemäß Anspruch 1, **dadurch gekennzeichnet, daß** man 2-(1-Chlorcyclopropyl)-3-(2-Chlor-phenyl)-2-hydroxy-propyl-1-hydrazinium-sulfat der Formel

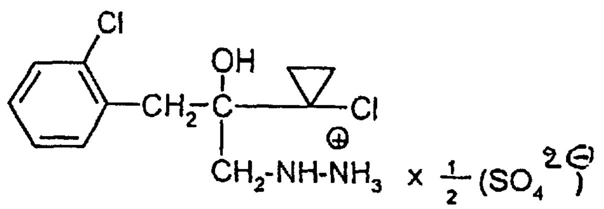

als Ausgangssubstanz einsetzt.

**3.** Verfahren gemäß Anspruch 1, **dadurch gekennzeichnet, daß** man bei der Durchführung der ersten Stufe Ammoniumthiocyanat als Reaktionskomponente einsetzt.

**4.** Verfahren gemäß Anspruch 1, **dadurch gekennzeichnet, daß** man bei der Durchführung der ersten Stufe bei Temperaturen zwischen 50°C und 120°C arbeitet.

**5.** Verfahren gemäß Anspruch 1, **dadurch gekennzeichnet, daß** man bei der Durchführung der zweiten Stufe bei Temperaturen zwischen 80°C und 120°C arbeitet.

**6.** Thiosemicarbazid-Derivate der Formel

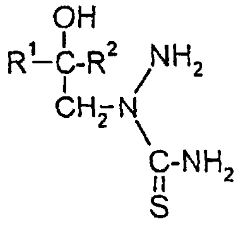

(IV)

in welcher
$R^1$ und $R^2$ die im Anspruch 1 angegebenen Bedeutungen haben.

**7.** Thiosemicarbazid-Derivat gemäß Anspruch 6, **gekennzeichnet durch** die Formel

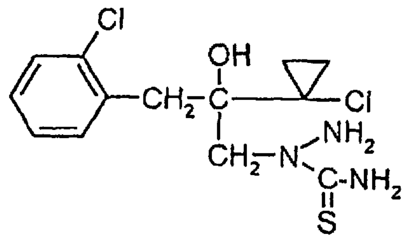

.

**8.** Verfahren zur Herstellung von Triazolinthion-Derivaten der Formel (I) gemäß Anspruch 1, **dadurch gekennzeichnet, daß** man

- 1-Chlor-2-hydroxy-ethan-Derivate der Formel

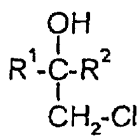

(V)

in welcher
$R^1$ und $R^2$ die oben angegebenen Bedeutungen haben,
mit Hydrazinhydrat gegebenenfalls in Gegenwart eines Verdünnungsmittels umsetzt und die dabei entstehenden Hydrazin-Derivate der Formel

(II) 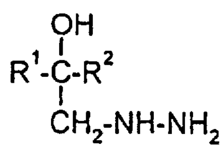

in welcher
$R^1$ und $R^2$ die oben angegebenen Bedeutungen haben,
gegebenenfalls mit Chlorwasserstoff oder Schwefelsäure in Gegenwart eines Verdünnungsmittels umsetzt,

- und die Hydrazin-Derivate der Formel (II) oder deren Additions-Salze mit Chlorwasserstoff oder Schwefelsäure ohne vorherige Isolierung mit Thiocyanat der Formel

Y-SCN (III)

in welcher

Y für Natrium, Kalium oder Ammonium steht,

in Gegenwart eines Verdünnungsmittels und gegebenenfalls in Gegenwart eines Katalysators umsetzt und

- die entstandenen Thiosemicarbazid-Derivate der Formel

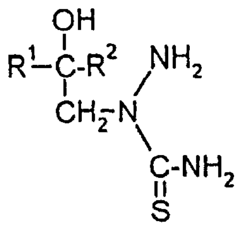

(IV)

in welcher
$R^1$ und $R^2$ die oben angegebenen Bedeutungen haben,
mit Ameisensäure, gegebenenfalls in Gegenwart eines Katalysators und gegebenenfalls in Gegenwart eines Verdünnungsmittels umsetzt.

**Claims**

**1.** Process for preparing triazolinethione derivatives of the formula

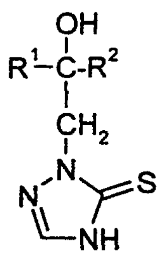

(I)

in which

$R^1$ represents straight-chain or branched alkyl having 1 to 4 carbon atoms, where these radicals may be mono- to tetrasubstituted by identical or different substituents from the group consisting of fluorine, chlorine, bromine, methoxy, ethoxy, propoxy, isopropoxy, alkoximino having 1 or 2 carbon atoms in the alkoxy moiety, cyclopropyl, cyclobutyl, cyclopentyl and cyclohexyl,
or
represents straight-chain or branched alkenyl having 2 to 5 carbon atoms, where each of these radicals may be mono- to trisubstituted by identical or different substituents from the group consisting of fluorine, chlorine, bromine, methoxy, ethoxy, propoxy, isopropoxy, cyclopropyl, cyclobutyl, cyclopentyl and cyclohexyl,
or
represents cycloalkyl having 3 to 6 carbon atoms, where each of these radicals may be mono- to trisubstituted by identical or different substituents from the group consisting of fluorine, chlorine, bromine, cyano, methyl, ethyl, propyl, isopropyl and tert-butyl,
or
represents phenylalkyl having 1 to 4 carbon atoms in the straight-chain or branched alkyl moiety, where the phenyl moiety may be mono- to trisubstituted by identical or different substituents from the group consisting of fluorine, chlorine, bromine, methyl, ethyl, tert-butyl, methoxy, ethoxy, methylthio, trifluoromethyl, trifluoromethoxy, trifluoromethylthio, chlorodifluoromethoxy, difluoromethoxy, chlorodifluoromethylthio, methoxycarbonyl, ethoxycarbonyl, methoximinomethyl, 1-methoximinoethyl, nitro and cyano,
or
represents phenylalkenyl having 2 to 4 carbon atoms in the alkenyl moiety, where the phenyl moiety may be mono- to trisubstituted by identical or different substituents from the group consisting of fluorine, chlorine, bromine, methyl, ethyl, tert-butyl, methoxy, ethoxy, methylthio, trifluoromethyl, trifluoromethoxy, trifluoromethylthio, chlorodifluoromethoxy, difluoromethoxy, chlorodifluoromethylthio, methoxycarbonyl, ethoxycarbonyl, methoximinomethyl, 1-methoximinoethyl, nitro and cyano,
or

represents phenoxyalkyl having 1 to 4 carbon atoms in the straight-chain or branched oxyalkyl moiety, where the phenyl moiety may be mono- to trisubstituted by identical or different substituents from the group consisting of fluorine, chlorine, bromine, methyl, ethyl, tert-butyl, methoxy, ethoxy, methylthio, trifluoromethyl, trifluoromethoxy, trifluoromethylthio, chlorodifluoromethoxy, difluoromethoxy, chlorodifluoromethylthio, methoxycarbonyl, ethoxycarbonyl, methoximinomethyl, 1-methoximinoethyl, nitro and cyano,
or
represents phenyl which may be mono- to trisubstituted by identical or different substituents from the group consisting of fluorine, chlorine, bromine, methyl, ethyl, tert-butyl, methoxy, ethoxy, methylthio, trifluoromethyl, trifluoromethoxy, trifluoromethylthio, chlorodifluoromethoxy, difluoromethoxy, chlorodifluoromethylthio, methoxycarbonyl, ethoxycarbonyl, methoximinomethyl, 1-methoximinoethyl, nitro and cyano,
and

$R^2$ represents straight-chain or branched alkyl having 1 to 4 carbon atoms, where these radicals may be mono- to tetrasubstituted by identical or different substituents from the group consisting of fluorine, chlorine, bromine, methoxy, ethoxy, propoxy, isopropoxy, alkoximino having 1 or 2 carbon atoms in the alkoxy moiety, cyclopropyl, cyclobutyl, cyclopentyl and/or cyclohexyl,
or
represents straight-chain or branched alkenyl having 2 to 5 carbon atoms, where each of these radicals may be mono- to trisubstituted by identical or different substituents from the group consisting of fluorine, chlorine, bromine, methoxy, ethoxy, propoxy, isopropoxy, cyclopropyl, cyclobutyl, cyclopentyl and cyclohexyl,
or
represents cycloalkyl having 3 to 6 carbon atoms, where each of these radicals may be mono- to trisubstituted by identical or different substituents from the group consisting of fluorine, chlorine, bromine, cyano, methyl, ethyl, propyl, isopropyl and tert-butyl,
or
represents phenylalkyl having 1 to 4 carbon atoms in the straight-chain or branched alkyl moiety, where the phenyl moiety may be mono- to trisubstituted by identical or different substituents from the group consisting of fluorine, chlorine, bromine, methyl, ethyl, tert-butyl, methoxy, ethoxy, methylthio, trifluoromethyl, trifluoromethoxy, trifluoromethylthio, chlorodifluoromethoxy, difluoromethoxy, chlorodifluoromethylthio, methoxycarbonyl, ethoxycarbonyl, methoximinomethyl, 1-methoximinoethyl, nitro and cyano,
or
represents phenylalkenyl having 2 to 4 carbon atoms in the alkenyl moiety, where the phenyl moiety may be mono- to trisubstituted by identical or different substituents from the group consisting of fluorine, chlorine, bromine, methyl, ethyl, tert-butyl, methoxy, ethoxy, methylthio, trifluoromethyl, trifluoromethoxy, trifluoromethylthio, chlorodifluoromethoxy, difluoromethoxy, chlorodifluoromethylthio, methoxycarbonyl, ethoxycarbonyl, methoximinomethyl, 1-methoximinoethyl, nitro and cyano,
or
represents phenoxyalkyl having 1 to 4 carbon atoms in the straight-chain or branched oxyalkyl moiety, where the phenyl moiety may be mono- to trisubstituted by identical or different substituents from the group consisting of fluorine, chlorine, bromine, methyl, ethyl, tert-butyl, methoxy, ethoxy, methylthio, trifluoromethyl, trifluoromethoxy, trifluoromethylthio, chlorodifluoromethoxy, difluoromethoxy, chlorodifluoromethylthio, methoxycarbonyl, ethoxycarbonyl, methoximinomethyl, 1-methoximinoethyl, nitro and cyano,
or
represents phenyl which may be mono- to trisubstituted by identical or different substituents from the group consisting of fluorine, chlorine, bromine, methyl, ethyl, tert-butyl, methoxy, ethoxy, methylthio, trifluoromethyl, trifluoromethoxy, trifluoromethylthio, chlorodifluoromethoxy, difluoromethoxy, chlorodifluoromethylthio, methoxycarbonyl, ethoxycarbonyl, methoximinomethyl, 1-methoximinoethyl, nitro and cyano,

**characterized in that**

a) in a first step, hydrazine derivatives of the formula

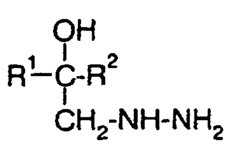

(II)

in which
$R^1$ and $R^2$ are each as defined above,
or their addition salts with hydrogen chloride or sulphuric acid,
are reacted with thiocyanate of the formula

Y-SCN (III)

in which
Y represents sodium, potassium or ammonium,
in the presence of a diluent and, if appropriate, in the presence of a catalyst, and

b) the resulting thiosemicarbazide derivatives of the formula

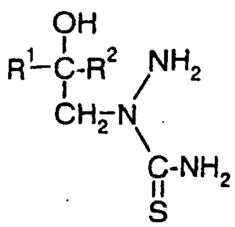

(IV)

in which
$R^1$ and $R^2$ are each as defined above

are reacted with formic acid, if appropriate in the presence of a catalyst and if appropriate in the presence of a diluent.

**2.** Process according to Claim 1, **characterized in that** the starting material used is 2-(1-chloro-cyclopropyl)-3-(2-chloro-phenyl)-2-hydroxy-propyl-1-hydrazinium sulphate of the formula

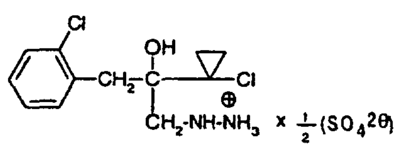

**3.** Process according to Claim 1, **characterized in that** the reaction component used for carrying out the first step is ammonium thiocyanate.

**4.** Process according to Claim 1, **characterized in that** the first step is carried out at temperatures between 50°C and 120°C.

**5.** Process according to Claim 1, **characterized in that** the second step is carried out at temperatures between 80°C and 120°C.

**6.** Thiosemicarbazide derivatives of the formula

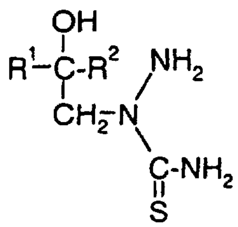 (IV)

in which
$R^1$ and $R^2$ are each as defined in Claim 1.

**7.** Thiosemicarbazide derivatives according to Claim 6, **characterized by** the formula

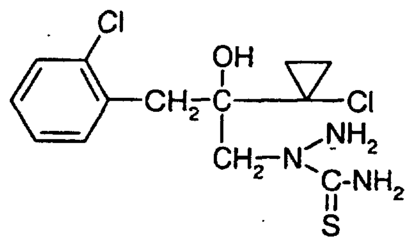

**8.** Process for preparing triazolinethione derivatives of the formula (I) according to Claim 1, **characterized in that**

- 1-chloro-2-hydroxy-ethane derivatives of the formula

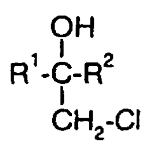 (V)

in which
$R^1$ and $R^2$ are each as defined above
are reacted with hydrazine hydrate, if appropriate in the presence of a diluent, and the resulting hydrazine derivatives of the formula

(II) 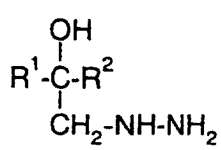

in which
$R^1$ and $R^2$ are each as defined above
are, if appropriate, reacted with hydrogen chloride or sulphuric acid in the presence of a diluent,

- and the hydrazine derivatives of the formula (II) or their addition salts with hydrogen chloride or sulphuric acid are reacted without prior isolation with thiocyanate of the formula

Y-SCN (III)

in which
Y represents sodium, potassium or ammonium
in the presence of a diluent and, if appropriate, in the presence of a catalyst, and

- the resulting thiosemicarbazide derivatives of the formula

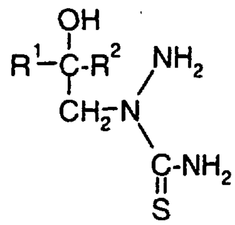

(IV)

in which
$R^1$ and $R^2$ are each as defined above
are reacted with formic acid, if appropriate in the presence of a catalyst and if appropriate in the presence of a diluent.

## Revendications

1. Procédé de production de dérivés de triazolinethione de formule

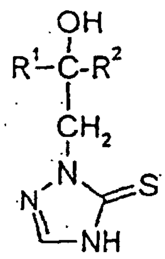

(I)

dans laquelle

$R^1$ est un reste alkyle linéaire ou ramifié ayant 1 à 4 atomes de carbone, les restes ainsi définis pouvant porter 1 à 4 substituants, identiques ou différents, fluoro, chloro, bromo, méthoxy, éthoxy, propoxy, isopropoxy, alkoximino ayant 1 ou 2 atomes de carbone dans la partie alkoxy, cyclopropyle, cyclobutyle, cyclopentyle et/ou cyclohexyle,
ou bien
un reste alcényle linéaire ou ramifié ayant 2 à 5 atomes de carbone, chacun des restes ainsi définis pouvant porter 1 à 3 substituants, identiques ou différents, fluoro, chloro, bromo, méthoxy, éthoxy, propoxy, isopropoxy, cyclopropyle, cyclobutyle, cyclopentyle et/ou cyclohexyle,
ou bien
un reste cycloalkyle ayant 3 à 6 atomes de carbone, chacun des restes ainsi définis pouvant porter 1 à 3 substituants, identiques ou différents, fluoro, chloro, bromo, cyano, méthyle, éthyle, propyle, isopropyle et/ou tertio-butyle,
ou bien
un reste phénylalkyle ayant 1 à 4 atomes de carbone dans la partie alkyle linéaire ou ramifiée, la partie phényle pouvant porter un à trois substituants, identiques ou différents, fluoro, chloro, bromo, méthyle, éthyle, tertio-butyle, méthoxy, éthoxy, méthylthio, trifluorométhyle, trifluorométhoxy, trifluorométhylthio, chlorodifluorométhoxy, difluorométhoxy, chlorodifluorométhylthio, méthoxycarbonyle, éthoxycarbonyle, méthoximinométhyle, 1-méthoximinoéthyle, nitro et/ou cyano,

ou bien
un reste phénylalcényle ayant 2 à 4 atomes de carbone dans la partie alcényle, la partie phényle pouvant porter un à trois substituants, identiques ou différents, fluoro, chloro, bromo, méthyle, éthyle, tertio-butyle, méthoxy, éthoxy, méthylthio, trifluorométhyle, trifluorométhoxy, trifluorométhylthio, chlorodifluorométhoxy, difluorométhoxy, chlorodifluorométhylthio, méthoxycarbonyle, éthoxycarbonyle, méthoximinométhyle, 1-méthoximinoéthyle, nitro et/ou cyano,
ou bien
un reste phénoxyalkyle ayant 1 à 4 atomes de carbone dans la partie oxyalkyle linéaire ou ramifiée, la partie phényle pouvant porter un à trois substituants, identiques ou différents, fluoro, chloro, bromo, méthyle, éthyle, tertio-butyle, méthoxy, éthoxy, méthylthio, trifluorométhyle, trifluorométhoxy, trifluorométhylthio, chlorodifluorométhoxy, difluorométhoxy, chlorodifluorométhylthio, méthoxycarbonyle, éthoxycarbonyle, méthoximinométhyle, 1-méthoximinoéthyle, nitro et/ou cyano,
ou bien
un reste phényle qui peut porter un à trois substituants, identiques ou différents, fluoro, chloro, bromo, méthyle, éthyle, tertio-butyle, méthoxy, éthoxy, méthylthio, trifluorométhyle, trifluorométhoxy, trifluorométhylthio, chlorodifluorométhoxy, difluorométhoxy, chlorodifluorométhylthio, méthoxycarbonyle, éthoxycarbonyle, méthoximinométhyle, 1-méthoximinoéthyle, nitro et/ou cyano,
et

$R^2$ représente un reste alkyle linéaire ou ramifié ayant 1 à 4 atomes de carbone, les restes ainsi définis pouvant porter un à quatre substituants, identiques ou différents, fluoro, chloro, bromo, méthoxy, éthoxy, propoxy, isopropoxy, alkoximino ayant 1 ou 2 atomes de carbone dans la partie alkoxy, cyclopropyle, cyclobutyle, cyclopentyle et/ou cyclohexyle,
ou bien
un reste alcényle linéaire ou ramifié ayant 2 à 5 atomes de carbone, chacun des restes ainsi définis pouvant porter un à trois substituants, identiques ou différents, fluoro, chloro, bromo, méthoxy, éthoxy, propoxy, isopropoxy, cyclopropyle, cyclobutyle, cyclopentyle et/ou cyclohexyle,
ou bien
un reste cycloalkyle ayant 3 à 6 atomes de carbone, chacun des restes ainsi définis pouvant porter un à trois substituants, identiques ou différents, fluoro, chloro, bromo, cyano, méthyle, éthyle, propyle, iso-propyle et/ou tertio-butyle,
ou bien
un reste phénylalkyle ayant 1 à 4 atomes de carbone dans la partie alkyle linéaire ou ramifiée, la partie phényle pouvant porter un à trois substituants, identiques ou différents, fluoro, chloro, bromo, méthyle, éthyle, tertio-butyle, méthoxy, éthoxy, méthylthio, trifluorométhyle, trifluorométhoxy, trifluorométhylthio, chlorodifluorométhoxy, difluorométhoxy, chlorodifluorométhylthio, méthoxycarbonyle, éthoxycarbonyle, méthoximinométhyle, 1-méthoximinoéthyle, nitro et/ou cyano,
ou bien
un reste phénylalcényle ayant 2 à 4 atomes de carbone dans la partie alcényle, la partie phényle pouvant porter un à trois substituants, identiques ou différents, fluoro, chloro, bromo, méthyle, éthyle, tertio-butyle, méthoxy, éthoxy, méthylthio, trifluorométhyle, trifluorométhoxy, trifluorométhylthio, chlorodifluorométhoxy, difluorométhoxy, chlorodifluorométhylthio, méthoxycarbonyle, éthoxycarbonyle, méthoximinométhyle, 1-méthoximinoéthyle, nitro et/ou cyano,
ou bien
un reste phénoxyalkyle ayant 1 à 4 atomes de carbone dans la partie oxyalkyle linéaire ou ramifiée, la partie phényle pouvant porter un à trois substituants, identiques ou différents, fluoro, chloro, bromo, méthyle, éthyle, tertio-butyle, méthoxy, éthoxy, méthylthio, trifluorométhyle, trifluorométhoxy, trifluorométhylthio, chlorodifluorométhoxy, difluorométhoxy, chlorodifluorométhylthio, méthoxycarbonyle, éthoxycarbonyle, méthoximinométhyle, 1-méthoximinoéthyle, nitro et/ou cyano,
ou bien
un reste phényle qui peut porter un à trois substituants, identiques ou différents, fluoro, chloro, bromo, méthyle, éthyle, tertio-butyle, méthoxy, éthoxy, méthylthio, trifluorométhyle, trifluorométhoxy, trifluorométhylthio, chlorodifluorométhoxy, difluorométhoxy, chlorodifluorométhylthio, méthoxycarbonyle, éthoxycarbonyle, méthoximinométhyle, 1-méthoximinoéthyle, nitro et/ou cyano,

**caractérisé en ce que** :

a) dans une première étape, on fait réagir des dérivés d'hydrazine de formule

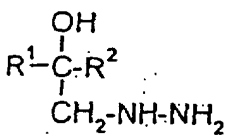

(II)

dans laquelle
$R^1$ et $R^2$ ont les définitions indiquées ci-dessus,
ou leurs sels d'addition avec le chlorure d'hydrogène ou l'acide sulfurique,
avec un thiocyanate de formule

$$Y\text{-}SCN \qquad (III)$$

dans laquelle
Y représente le sodium, le potassium ou l'ion ammonium, en présence d'un diluant et en présence éventuelle d'un catalyseur, et
b) on fait réagir les dérivés de thiosemicarbazide produits de formule

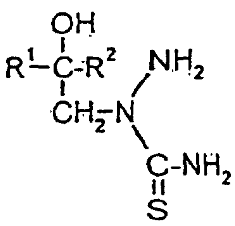

(IV)

dans laquelle
$R^1$ et $R^2$ ont les définitions indiquées ci-dessus,
avec l'acide formique, éventuellement en présence d'un catalyseur et en présence éventuelle d'un diluant.

2. Procédé suivant la revendication 1, **caractérisé en ce qu'**on utilise comme substance de départ le sulfate de 2-(1-chlorocyclopropyl)-3-(2-chlorophényl)-2-hydroxypropyl-1-hydrazinium de formule

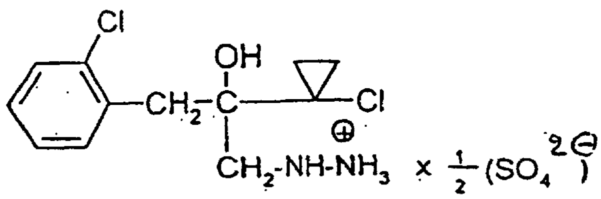

3. Procédé suivant la revendication 1, **caractérisé en ce qu'**on utilise le thiocyanate d'ammonium comme composant réactionnel dans la conduite de la première étape.

4. Procédé suivant la revendication 1, **caractérisé en ce qu'**on opère à des températures comprises entre 50°C et 120°C dans la conduite de la première étape.

5. Procédé suivant la revendication 1, **caractérisé en ce qu'**on opère à des températures comprises entre 80°C et 120°C dans la conduite de la seconde étape.

6. Dérivés de thiosemicarbazide de formule

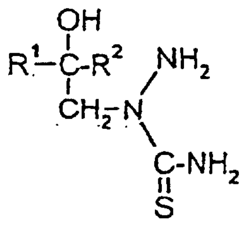 (IV)

dans laquelle
$R^1$ et $R^2$ ont les définitions indiquées dans la revendication 1.

**7.** Dérivé de thiosemicarbazide suivant la revendication 6, **caractérisé par** la formule

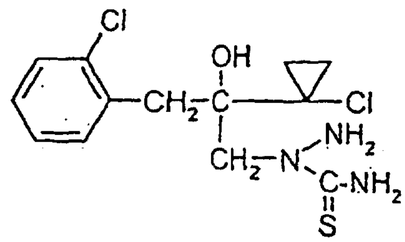

**8.** Procédé de production de dérivés de triazolinethione de formule (I) suivant la revendication 1, **caractérisé en ce que** :

- on fait réagir des dérivés de 1-chloro-2-hydroxy-éthane de formule

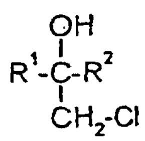 (V)

dans laquelle
$R^1$ et $R^2$ ont les définitions indiquées ci-dessus,
avec l'hydrate d'hydrazine, le cas échéant en présence d'un diluant, et on fait réagir les dérivés d'hydrazine ainsi produits de formule

(II) 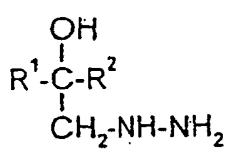

dans laquelle
$R^1$ et $R^2$ ont les définitions indiquées ci-dessus, le cas échéant avec le chlorure d'hydrogène ou l'acide sulfurique en présence d'un diluant,

- et on fait réagir les dérivés d'hydrazine de formule (II) ou leurs sels d'addition avec le chlorure d'hydrogène

ou l'acide sulfurique
sans isolement préalable, avec un thiocyanate de formule

Y-SCN (III)

dans laquelle
Y désigne le sodium, le potassium ou l'ion ammonium, en présence d'un diluant et en présence éventuelle d'un catalyseur, et

- on fait réagir les dérivés de thiosemicarbazide produits de formule

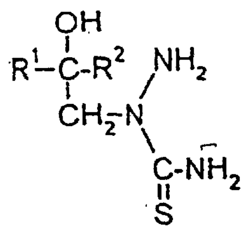

(IV)

dans laquelle
$R^1$ et $R^2$ ont les définitions indiquées ci-dessus,
avec l'acide formique, le cas échéant en présence d'un catalyseur et en présence éventuelle d'un diluant.